# EUROPEAN PATENT APPLICATION

(11) **EP 2 873 662 A1**
(43) Date of publication of application: **20.05.2015**
(21) Application number: 13005382.0
(22) Date of filing: 15.11.2013
(51) Int. Cl.: C07D 333/24, C08G 61/12, H01L 51/00

(54) **Star-shaped compounds for dye-sensitized solar cells**

(71) Applicant: Heraeus Precious Metals GmbH & Co. KG, 63450 Hanau (DE)
(72) Inventor: Kausch-Busies, Nina, 51467 Bergisch Gladbach (DE); Ponomarenko, Sergei A., Moskau 107589 (RU); Solodukhin, Alexander, Zheleznogorsk; Kursk region; 307170 Moskau (RU); Luponosov, Juriy N., 117628 Moskau (RU)
(74) Representative: Herzog, Martin

(57) **Abstract**

The present invention relates to compounds of the general formula (I)

K⁅L-R]ₙ (I)

The present invention also relates to electronic components comprising at least one semiconducting layer, wherein the semiconducting layer comprises these compounds, and to the use of these compounds for the production of semiconducting layers in electronic components.

## Description

The present invention relates to compounds of the general formula (I)

K⁅L-R]ₙ (I),

to electronic components, in particular to dye-sensitized solar cells, comprising at least one semiconducting layer, wherein the semiconducting layer comprises these compounds, and to the use of these compounds for the production of semiconducting layers in electronic components, in particular in dye-sensitized solar cells.

A solar cell (also called photovoltaic cell) is a photoelectrical device that converts the energy of sunlight into electricity. According to materials of cells and operation theory, it can be divided into crystalline silicon solar cell, amorphous silicon solar cell, copper indium gallium selenite (CIGS) solar cell, cadmium telluride (CdTe) thin film solar cell, silicon thin film solar cell and dye-sensitized solar cell (DSSC), etc., wherein DSSC has advantages of simplifying the fabrication process, mass production and low material cost, so that it already becomes one of the important next-generation solar cells.

In general, a unit cell of a dye-sensitized solar cell consists of upper and lower transparent substrates, a conductive transparent electrode consisting of transparent conductive oxide (TCO) respectively formed on the surface of the transparent substrates, a dye-adsorbed transition metal oxide porous layer, usually based on TiO₂, formed on one conductive transparent electrode corresponding to a first electrode, a thin film electrode formed on the other conductive transparent electrode corresponding to a second electrode, and electrolyte filled between the first and the second electrode.

As the dye for the dye-sensitized solar cell, a ruthenium metal complex exhibiting high photoelectric conversion efficiency of 10% or more has been widely used, but the ruthenium metal complex is too expensive and difficult to purify. However, it has been found that a metal-free organic dye exhibiting excellent properties of absorption efficiency, redox reaction stability and charge-transfer (CT) absorption can be used as a dye for a solar cell replacing the expensive ruthenium metal complex.

Organic dyes that are suitable for DSSCs usually are chromophores comprising a donor function, an acceptor function, an anchor group by means of which the dye can be adsorbed to the transition metal porous layer and which is arranged in the proximity of the acceptor function, and a hydrophobic periphery. Dyes having such a structure are, for example, disclosed by Mishra et al. in Angewandte Chemie, Vol. 121 (14), pages 2510-2536 (2009). Some of these compounds comprise triphenylamine as the donor function. However, compounds based in triphenylamine that are described by Mishra *et al.* are characterized by a low symmetry.

Dyes having a higher degree of symmetry and which can be used for the preparation of semiconducting layers in solar cells are disclosed in WO-A-2012/100908. These compounds, however, have the disadvantage that their absorption to porous transition metal oxides such as TiO₂ is sometimes to low, which impedes their use as a dye in a dye-sensitized solar cell.

The present invention was therefore based on the object of overcoming the disadvantages resulting from the prior art in connection with the use of organic oligomeric compounds in solar cells, in particular in dye-sensitized solar cells.

In particular, the present invention was based on the object of providing organic oligomeric compounds which not only are distinguished by particularly advantageous electronic and optical properties and by a high absorption of sunlight, but which moreover also have a particularly good processability, in particular a good wet processability. Layers produced from such compounds should be characterized by a particularly advantageous photoactivity.

Moreover, the organic oligomeric compounds should be characterized by an improved binding affinity to porous transition metal oxides such as TiO₂, compared to the compounds of the prior art, making them particularly suitable as dyes in a dye-sensitized solar cell.

A contribution towards achieving the abovementioned objects is made by compounds of the general formula (I)

K⁅L-R]ₙ (I)

wherein
- n: is an integer from 3 to 6, where n can assume in particular the value 3, 4, 5 or 6,
- R: represents H or a non-conjugated chain,
- L: are linear conjugated units according to the general formula (II) 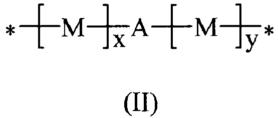 in which
x, y in each case independently of each other represent an integer from 0 to 20, particularly preferably from 0 to 10 and moreover preferably from 0 to 5, but very particularly preferably independently of each other represent 0, 1 or 2,
A represents an acceptor group selected from the group consisiting of group IIIa-IIIl 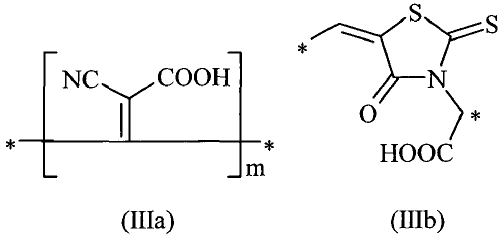 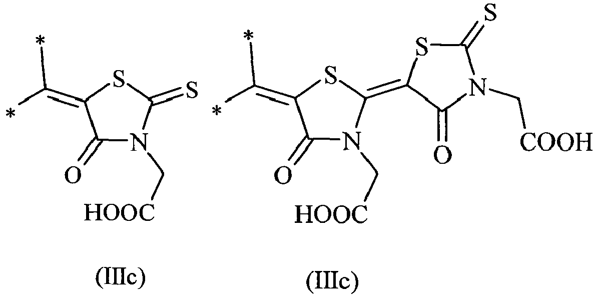 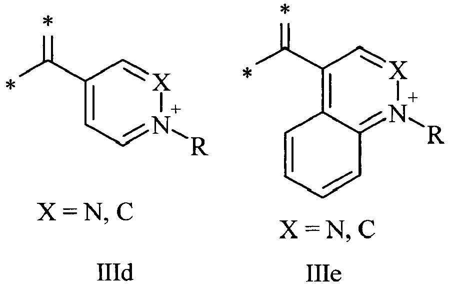 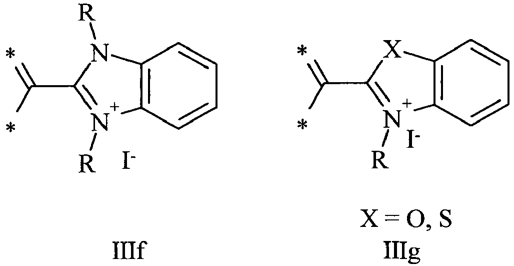 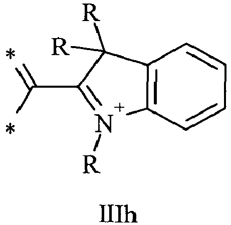 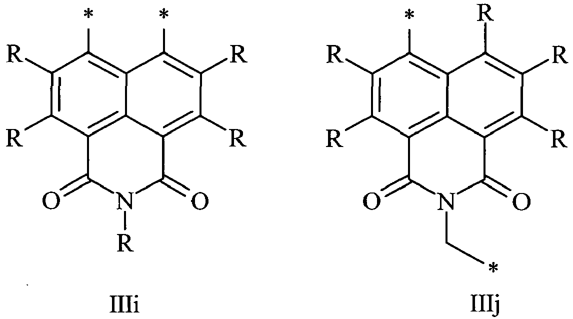 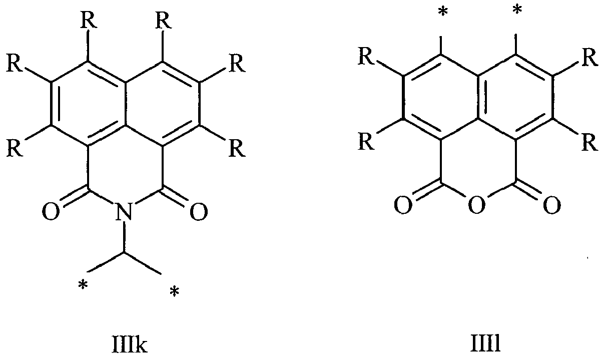 in which
R represents H or a linear or branched C₁-C₂₀-alkyl group, preferably a C₁-C₁₂-alkyl group, or a linear C₁-C₂₀-alkyl group, preferably C₁-C₁₂-alkyl group, which is optionally interrupted by one or more O or S atoms or silylene, phosphonoyl or phosphoryl groups, where, if the aryl compound A comprises two radicals R³, these can be identical or different, and
m represents an integer from 1 to 20, particularly preferably from 1 to 10 and most preferably from 1 to 5, and wherein in case of group IIIa the NC-group may be partially or completely hydrolyzed to form a H₂N-C(O)-group or a OH-C(O)-group and the -COOH-group may be esterified to form a -COOR¹-group, in which R¹ represents a C₁-C₁₀-alkyl group,
M represents an aryl compound according to one of the following formulae IVa-IVq 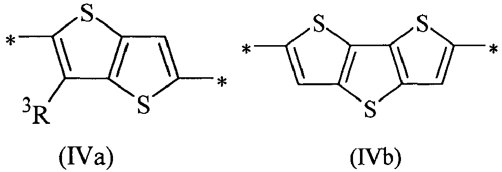 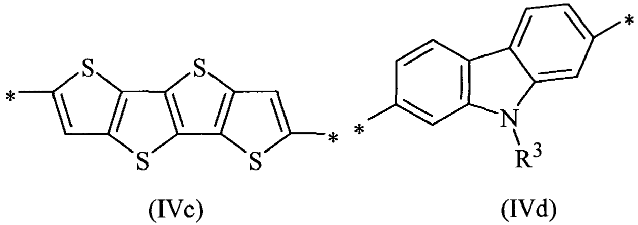 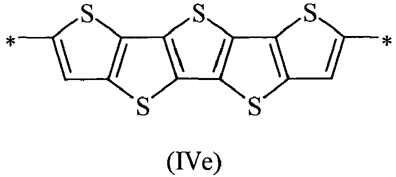 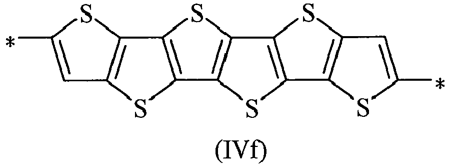 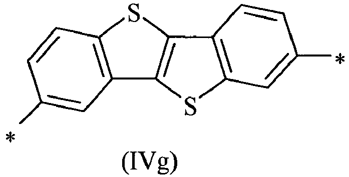 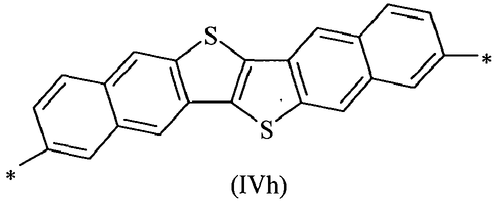 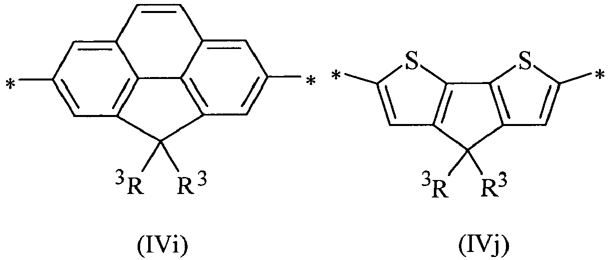 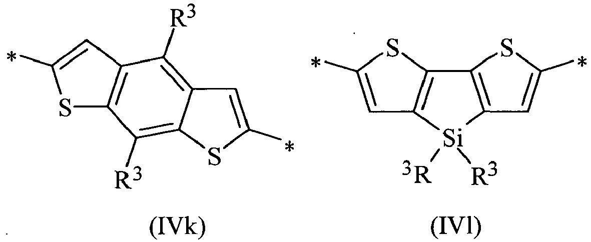 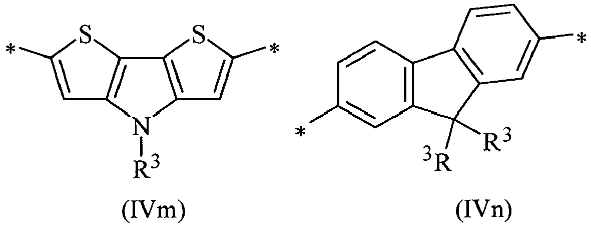 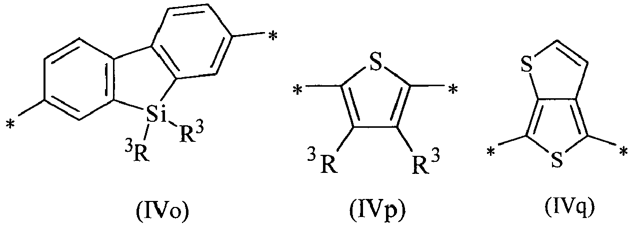 in which
R³ represents H or a linear or branched C₁-C₂₀-alkyl group, preferably a C₁-C₁₂-alkyl group, or a linear C₁-C₂₀-alkyl group, preferably C₁-C₁₂-alkyl group, which is optionally interrupted by one or more O or S atoms or silylene, phosphonoyl or phosphoryl groups, where, if the aryl compound A comprises two radicals R³, these can be identical or different,
- K: represents a branching group according to one of the following formulae Va-Vi 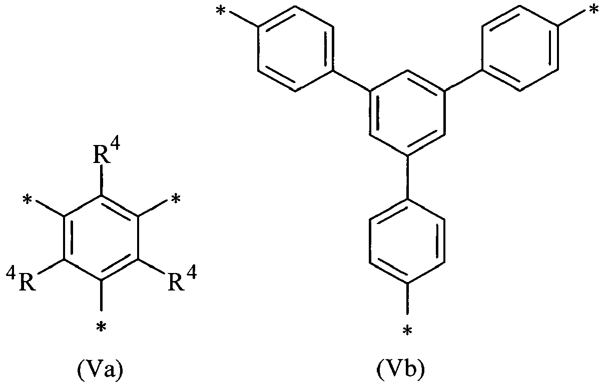 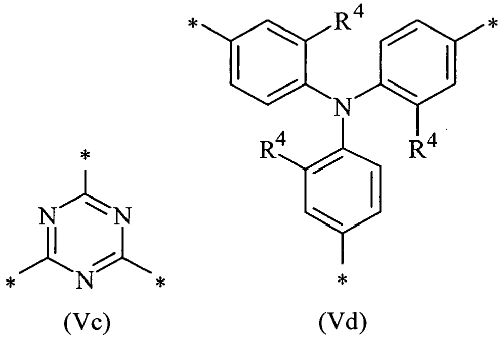 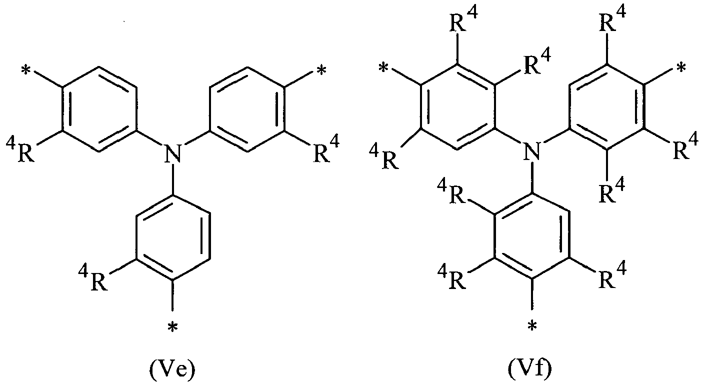 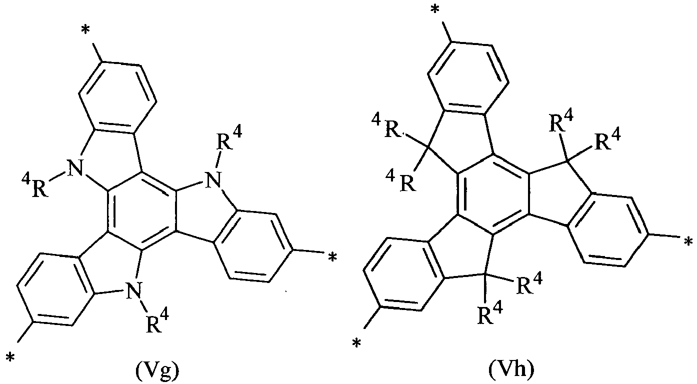 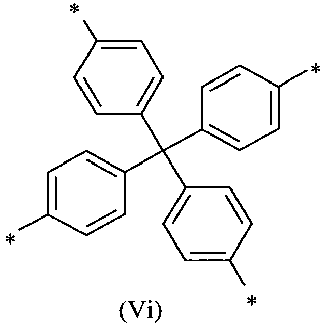 in which
R⁴ represents H or a linear or branched C₁-C₂₀-alkyl group, preferably a C₁-C₁₂-alkyl group, or a linear C₁-C₂₀-alkyl group, preferably C₁-C₁₂-alkyl group, which is optionally interrupted by one or more O or S atoms or silylene, phosphonoyl or phosphoryl groups.

The positions labelled with * in the formulae (II), (IIIa) and (IIIb), (IVa)-(IVq) and (Va)-(Vi) in each case identify the linkage sites. In the case of the formula (II), these are the bonding sites in which a linkage of the linear conjugated units L with the branching group K (via the structural element -[M]ₓ- if x > 0 or via the structural element -A- if x = 0) and the outer radical R (via the structural element -[M]_{y}- if y > 0 or via the structural element -A- if y = 0) takes place. In the case of the formulae (IIIa) and (IIIb), these are the bonding sites in which a linkage of the acceptor group A with the structural element -[M]ₓ- (if x > 0) or the branching group K (if x = 0) or the structural element -[M]_{y}- (if y > 0) or the radical R (if y = 0) takes place. In the case of the formulae (IVa)-(IVq), these are the bonding sites in which in the case of the structural element -[M]ₓ- a linkage of the unit M with the branching group K and with the acceptor group A or with a further unit M (if x > 1) takes place, and in the case of the structural element -[M]_{y}- a linkage of the unit M with the acceptor group A or a further unit M (if y > 1) and the radical R takes place. In the case of the formulae (Va)-(Vi), these are the bonding sites in which a linkage of the branching group K with the structural element -[M]ₓ- (if x > 0) or the acceptor group A (if x = 0) takes place.

The compounds according to the invention preferably have symmetric structure. The expression "*symmetric structure"* as used herein indicates that within a given compound of the general formula (I) all connecting points within a given connecting level (in the case of a branching group K with, for example, structural formula (Ve) these are the three branching points indicated with "*") are connected to the same molecule of the next level (in case of a branching group K with, for example, the structural formula (Ve) this means that each branching point indicated with "*" is connected to the same molecule L). However, this definition of symmetry does not apply for acceptor group "A" with formula (IIIa), if in a given compound of the general formula (I) in these acceptor groups the cyanide group is hydrolysed to a different degree. This means that if, for example, a compound comprises branching group (Ve) (three connecting points) and if these three connecting points are linked to the following three groups -L-R (x = 0, y = 0, A = group (IIIa), R = H), 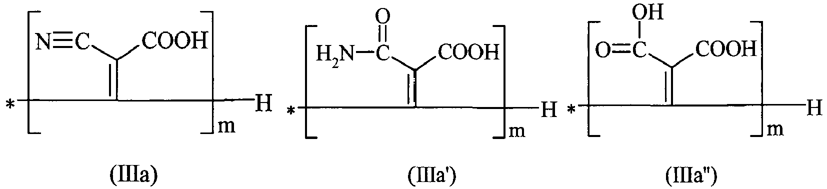 such a compound is nevertheless regarded as "*symmetrical"* as it is based on the same structural precursor-unit (i. e. formula (IIIa)). The same also applies if in case of the acceptor group "A" with formula (IIIa) some of the COOH-groups within a compound of the general formula (I) may be present in the form of an alkyl ester. Thus, if in case of n = 3 one or two of the three COOH-groups are esterified and are thus present in the form -COOR, in which R may be an alkyl-group such as a methyl- or an ethyl-group, the compound is also considered as "*symmetrical*".

The compounds according to the invention preferably also have a so-called *"core-shell structure",* in which the branching group K forms the core and the units -L-R bonded to the core form the shell. The compounds can in principle be oligomers or polymers.

In the context of the invention, the core-shell structure is a structure at the molecular level, i.e. it relates to the structure of a molecule as such.

The compounds according to the invention have, if n is, for example, 3, 4 or 6, a structure according to the following formulae (I-3), (I-4) or (I-6): 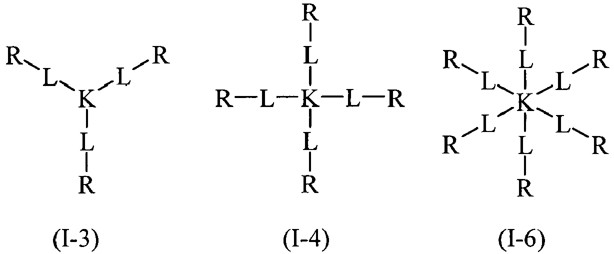 in which K, L and R have the abovementioned meaning.

The radical R is preferably a non-conjugated chain. Preferred non-conjugated chains are those which have a high flexibility, i.e. a high (intra)molecular mobility, as a result interact readily with solvent molecules and thus generate an improved solubility. In the context of the invention, flexible is to be understood in the sense of (intra)molecularly mobile. The non-conjugated chains (R) are straight-chain or branched aliphatic, unsaturated or araliphatic chains which have 1 to 20 carbon atoms, preferably which have 1 to 12 carbon atoms, and are optionally interrupted by oxygen, or C₃-C₈-cycloalkylenes. Aliphatic and oxyaliphatic groups, i.e. alkoxy groups, or straight-chain or branched aliphatic groups, in particular C₁-C₂₀-alkyl groups, interrupted by one or more oxygen or sulphur atoms or silylene, phosphonyl or phosphoryl groups, are preferred. Linear or branched C₁-C₂₀-alkyl groups, in particular C₁-C₁₂-alkyl groups, are particularly preferred according to the invention as radicals R. Examples of suitable radicals R are alkyl groups, such as n-hexyl, n-heptyl, n-octyl, n-nonyl, n-decyl and n-dodecyl groups, and alkoxy groups, such as n-hexyloxy, n-heptyloxy, n-octyloxy, n-nonyloxy, n-decyloxy and n-dodecyloxy groups, or C₃-C₈-cycloalkylenes, such as cyclopentyl, cyclohexyl or cycloheptyl. Particularly preferred residues R are H, -CH₃ and -C₂H₅.

It is preferred that in the compounds according to the present invention the structural elements M in the linearly conjugated chains L comprise optionally substituted 2,5-thienylene (IVp) 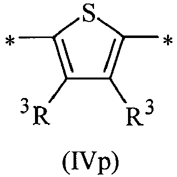 wherein the radicals R³ can be identical or different and represent H or a linear or branched C₁-C₂₀-alkyl group, preferably a C₁-C₁₂-alkyl group, or a linear C₁-C₂₀-alkyl group, preferably C₁-C₁₂-alkyl group, which is optionally interrupted by one or more O or S atoms or silylene, phosphonoyl or phosphoryl groups. In this connection, structural elements M in which R³ in each case represents a hydrogen atom are very particularly preferred. In this context, the structural units M can be present as monomers (in this case x and y have the value 1) 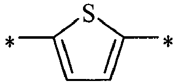 or as dimers (in this case x and y have the value 2)

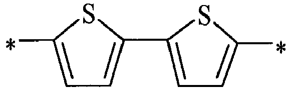

It is also preferred that in the compounds according to the present invention the branching group K represents a branching group of the formula (Vd) 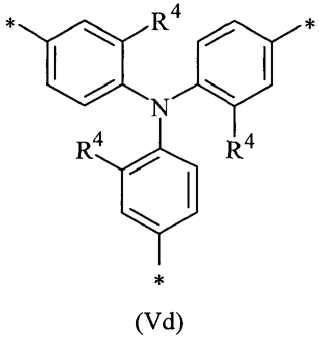 in which
- R⁴: represents H or -O- (CH₂)ₙCH₃, wherein n is an integer from 0 to 11, preferably from 0 to 8 and more preferably from 0 to 2.

According to a preferred embodiment of the compounds according to the invention,
- M: represents optionally substituted 2,5-thienylene of formula (IVp): 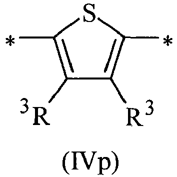 in which
R³ can be identical or different and represents H or a linear or branched C₁-C₂₀-alkyl group, preferably a C₁-C₁₂-alkyl group, or a linear C₁-C₂₀-alkyl group, preferably C₁-C₁₂-alkyl group, which is optionally in-terrupted by one or more O or S atoms or silylene, phosphonoyl or phosphoryl groups;
- K: represents a branching group of the formula (Vd) 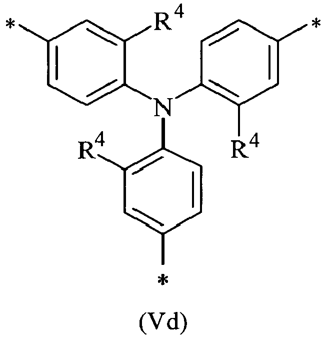 in which
R⁴ represents H or -O- (CH₂)ₙCH₃, wherein n is an integer from 0 to 11, preferably from 0 to 8 and more preferably from 0 to 2.
- x: represents 2;
- y: represents 0;
- A: represents an acceptor group of the formula (IIIa), (IIIa') or (IIIa") 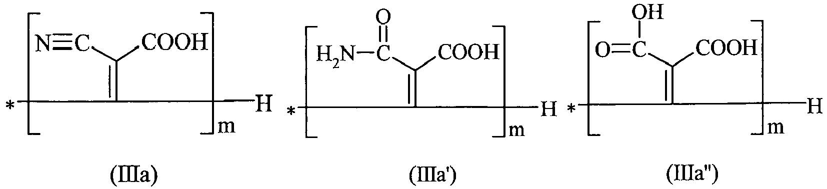 in which the -COOH-group may optionally be esterified to form a -COOR-group,
and
- R: represents a -C₂H₃ or a -CH₃ radical.

According to a particularly preferred embodiment of the compound according to the invention,
- M: represents 2,5-thienylene 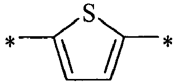
- K: represents triphenylamine 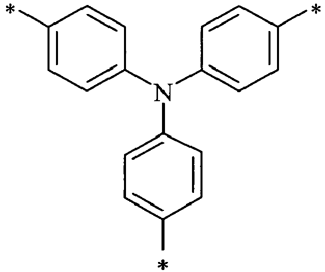
- x: represents 2;
- y: represents 0;
- A: represents an acceptor group of the formula (IIIa), (IIIa') or (IIIa") 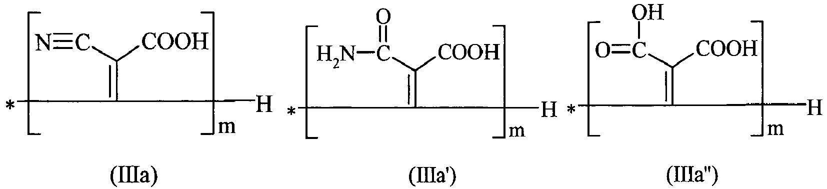 in which the -COOH-group may optionally be esterified to form a -COOR-group,
and
- R: represents a -C₂H₃ or a -CH₃ radical.

Layers of the compounds of the general formula (I) according to the invention are preferably conductive or semiconducting. The invention particularly preferably provides those layers of the compounds or mixtures which are semiconducting. Those layers of the compounds which have a mobility for charge carriers of at least 10⁻⁴ cm²/Vs are particularly preferred. Charge carriers are e.g. positive hole charges.

The compounds according to the invention are typically readily soluble in the commonly available organic solvents and are therefore outstandingly suitable for processing from solution. Solvents which are suitable in particular are aromatics, ethers or halogenated aliphatic hydrocarbons, such as, for example, chloroform, toluene, benzene, xylenes, diethyl ether, methylene chloride, chlorobenzene, dichlorobenzene or tetrahydrofuran, or mixtures of these. The compounds according to the invention are conventionally soluble in these solvents to the extent of at least 0.01 wt.%, preferably at least 0.1 wt.%, more preferably at least 1 wt.%, particularly preferably at least 5 wt.%. The compounds according to the invention form high quality layers of uniform thickness and morphology from evaporated solutions and are therefore suitable for electronic uses, in particular as a semiconductor layer in organic solar cells.

The compounds according to the present invention can be prepared by means of a process wherein
- the -[L-R] radical or radicals or synthesis precursors of the -[L-R] radical or radicals are present as an organoboron compound and the branching group K is present as an aryl or heteroaryl halide, or
- the -[L-R] radical or radicals or synthesis precursors of the -[L-R] radical or radicals are present as an aryl or heteroaryl halide and the branching group K is present as an organoboron compound,
and the -[L-R] radical or radicals or synthesis precursors of the -[L-R] radical or radicals are bonded to the branching group K via a Suzuki coupling.

This process is described in detail in WO-A-2012/100908 and the disclosure of this reference with respect to the preperation of compounds of the general formula (I) is incorporated herein by reference.

A contribution towards achieving the abovementioned objects is also made by an electronic component comprising at least one semiconducting layer, wherein the semiconducting layer comprises the compound according to the present invention.

According to a preferred embodiment of the electronic component according to the present invention, the electronic component is a solar cell, in particular a dye-sensitized solar cell. In this context it is preferred that the dye-sensitized solar cell comprises
(i) a first electrode having a porous oxide semiconductor layer coated onto a transparent conductive substrate, wherein the compound according to the present invention is immobilized on the porous oxide semiconductor layer,
(ii) a second electrode which is provided so as to face the porous oxide semiconductor layer of the first electrode,
(iii) a redox electrolyte disposed between the first electrode and the second electrode.

The dye-sensitized solar cell according to the present invention comprises as a first component (i) a first electrode having a porous oxide semiconductor layer coated onto a transparent conductive substrate, wherein the compound according to the present invention is immobilized on the porous oxide semiconductor layer.

As the transparent conductive substrate onto which the porous oxide semiconductor layer is applied preferably a transparent substrate is used having a conductive surface. Specific examples thereof may include those obtained by forming a thin film of metals such as steel, silver, gold, etc. or conductive metal oxide such as indium, fluorine or antimony tin oxide, in particular indium tin oxide (ITO), on the surface of transparent materials such as glass or a transparent polymer material such as polyethyleneterephthalate or polyethersulfone, etc. The conductivity may be preferably 1000 Ω or less, particularly 100 Ω or less.

The porous layer of the oxide semiconductor preferably comprises particles of the oxide semiconductor. Specific examples thereof may include oxides of titanium, tin, zinc, tungsten, zirconium, gallium, indium, yttrium, niobe, tantalum, vanadium, etc. Zinc oxide and tin oxide are more preferable, and titanium oxide (TiO₂) is most preferable. The average diameter of the oxide semiconductor particle may be preferably 1 - 500 nm, more preferably 1 - 500 nm.

The oxide semiconductor thin film may be directly formed on the transparent conductive substrate for example by spraying oxide semiconductor particles, or it may be formed by a method of electrically precipitating a semiconductor particle thin film onto the transparent conductive substrate with the substrate as an electrode, or by a method of coating a paste containing particles obtainable by hydrolysis of semiconductor particle precursors such as semiconductor alkoxides or semiconductor particle slurries onto the transparent conductive substrate, and then subjecting the coated substrate to a heat-treatment. In this method, the slurry may be obtained by dispersing coagulated oxide semiconductor particles in a dispersion medium by a common method such that the average primary particle diameter may become 1 - 200 nm. The dispersion medium for dispersing the slurry may include those capable of dispersing semiconductor particles without specific limitation, and specific examples thereof may include water, alcohol such as ethanol, etc., ketone such as acetone, acetylacetone, etc., hydrocarbon such as hexane, etc. and a combination thereof. Water is preferable because it minimizes viscosity change of the slurry. A dispersion stabilizer may be used to stabilize the dispersion of the oxide semiconductor particle. Specific examples of the dispersion stabilizer may include acids such as acetic acid, hydrochloric acid, nitric acid, etc., acetylacetone, acrylic acid, polyethyleneglycol, polyvinylacohol, etc..

The transparent conductive substrate coated with the slurry may be heat treated to a temperature of 100°C or more, preferably 200°C or more, and the upper limit may be generally a melting point of the substrate, commonly 900°C, preferably 600°C. The heating time may be preferably within 4 hours, but is not specifically limited thereto. The thickness of the thus obtained thin film on the transparent conductive substrate may be 1 - 200 µm, preferably 1 - 200 µm.

The thus obtained oxide semiconductor thin film may be secondarily treated. For example, the thin film may be immersed in a solution of alkoxide, chloride, nitride, sulfide, etc. of the same metal as the semiconductor, and heat-treating, thereby improving the performance of the semiconductor thin film. The metal alkoxide may include titanium ethoxide, titanium isopropoxide, titanum t-butoxide etc., these alkoxides preferably being applied in the form of solutions in which the corresponding alcohol serves as the solvent. The chloride may include titanium tetrachloride, tin tetrachloride, zinc chloride, etc., wherein the chlorides are preferably applied in the form of aqueous solutions.

The thus obtained oxide semiconductor thin film consists of oxide semiconductor particles and the compound according to the present invention is immobilized onto the porous oxide semiconductor layer, in particular onto the surface of the oxide semiconductor particles. In the case of TiO₂-particles the compound according to the present invention is bound to the TiO₂-particles via the COOH-group of the acceptor group.

The method of immobilizing the compound according to the present invention onto the oxide semiconductor particle thin film, in particular onto the surface of the oxide semiconductor particles, is not specifically limited. For example, the conductive transparent substrate, on which the oxide semiconductor thin film is formed, may be immersed in a solution or dispersion containing the compound according to the present invention. The solution can be obtained by dissolving the compound in a solvent capable of dissolving it and the dispersion can be obtained by dispersing the compound in a dispersing agent capable of dispersing it. The concentration of the compound in the solution or dispersion may be appropriately determined. The immersion temperature may be room temperature to a boiling point of the solvent or dispersing agent, and the immersion time may be 1 minute to 48 hours. The solvent or dispersing agent for the compound according to the present invention may include water, tetrahydrofuran, 1,2-dichlorethane, methanol, ethanol, acetonitrile, dimethylsulfoxide, dimethylformamide, acetone, t-butanol or a mixture of at least two of these solvents. The concentration of the compound in the solution or dispersion may be in the range from 1 × 10⁻⁶ M to 1 M, preferably from 1 × 10⁻⁵ M to 0.1 M.

The dye-sensitized solar cell according to the present invention comprises as a second component (ii) a second electrode (counter electrode) facing the porous oxide semiconductor layer of the first electrode. For this purpose, materials can be used having a sufficiently high conductivity and being able to catalyze the reduction of the redox electrolyte. Suitable as the counter electrode are, for example, glass substrates or polymer films onto which conductive materials such as platinum, carbon, rhodium, ruthenium, etc. have been deposited.

The dye-sensitized solar cell according to the present invention comprises as a third component (iii) a redox electrolyte disposed between the first electrode (i) and the second electrode (iii). The redox electrolyte may include halogen redox electrolyte comprising a halogen compound with a halogen ion as a counter ion and a halogen molecule, metal redox electrolyte such as metal complex such as ferrocyanate salt-ferrocyanate salt or ferrocene-ferricinium ion, cobalt complex, etc., organic redox electrolyte such as alkylthiol-alkyidisulfide, viologen dyes, hydroquinone-quinone, etc., wherein a halogen-based redox electrolyte is preferable. The halogen-based redox electrolyte comprises a halogen compound and a halogen molecule, the halogen molecule may preferably be an iodine molecule (I₂), and the halogen compound with a halogen ion as a counter ion may include metal halides such as LiI, NaI, KI, CaI₂, MgI₂, CuI, etc., an organic ammonium salt of halogen such as tetraalkylammonium iodide, imidazolium iodide, pyridium iodide, etc..

If the redox electrolyte is present in the form of a solution comprising the redox electrolyte and a solvent, an electrochemically inert solvent should be used. Specific examples thereof may include acetonitrile, propylenecarbonate, ethylene carbonate, 3-methoxypropionitrile, methoxyacetonitrile, ethyleneglycol, propylene glycol, diethylene glycol, triethylene glycol, butyrolactone, dimethoxyethane, dimethyl carbonate, 1,3-dioxolan, methylformate, 2-methyltetrahydrofuran, 3-methoxy-oxazolidin-2-on, sulfolane, tetrahydrofuran, water, etc., and acetonitrile, propylenecarbonate, ethylene carbonate, 3-methoxypropionitrile, ethyleneglycol, 3-methoxy-oxazolidin-2-on, butyrolactone, etc. are particularly preferred. The solvent may be used alone or in combination with another solvent. If the solid electrolyte is present in the form of a gel, gelling agent such as starch can be used serving as a matrix for the redox electrolyte.

The dye-sensitized solar cell according to the present invention can be manufactured by processes known to the person skilled in the art, for example by means of the process disclosed in EP-A-2 386 607. For example, the dye-sensitized solar cell can be manufactured by coating a titanium oxide paste on a conductive transparent substrate, heat-treating the paste-coated substrate to form a titanium oxide thin film, impregnating the substrate having the titanium oxide thin film with a solution or dispersion in which the compound according to the present invention is dissolved or dispersed so as to immobilize the compound onto the porous oxide semiconductor layer, in particular onto the surface of the SiO₂-particles, providing a counter electrode, for example glass coated with a conductive layer serving as the counter-electrode, such that the conductive layer faces the porous oxide semiconductor layer, and filling the space between the two electrodes with the redox electrolyte.

A contribution towards achieving the abovementioned objects is also made by the use of compounds according to the present invention for the production of semiconducting layers in electronic components. Particularly preferred is use of compounds according to the present invention for the production of semiconducting layers in a dye-sensitized solar cell, wherein the compounds are used as the dye.

The present invention is now explained in more detail with the aid of non-limiting figures and examples.

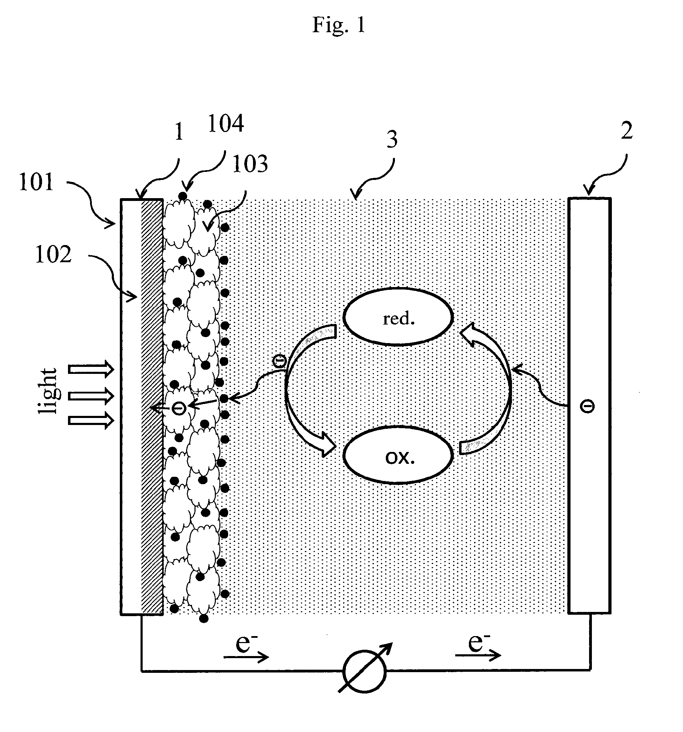

Figure 1 shows a dye-sensitized solar cell according to the present invention. The solar cell comprises a first electrode 1 having a porous oxide semiconductor layer 103, preferably based on TiO₂-particles, coated onto a transparent conductive substrate 101,102. The transparent conductive substrate 101,102 may for example be a transparent material 101 such as glass or a transparent polymer foil, for example a foil based on polyethylene terephthalate or polyethersulfone, onto which a transparent conductive coating 102, for example a layer of indium tin oxide (ITO), has been applied. The compound according to the present invention 104, for example compound **1** or compound **2** prepared in the examples below, is immobilized on the porous oxide semiconductor layer 103. The solar cell further comprises a second electrode 2, which is provided so as to face the porous oxide semiconductor layer 103 of the first electrode 1, and a redox electrolyte 3 that is disposed between the first electrode 1 and the second electrode 2.

If sunlight passes through the transparent electrode 1 into the layer comprising the compound according to the present invention 104, where it can excite electrons that then flow into the titanium dioxide 103. The electrons flow toward the transparent electrode 1 where they are collected for powering a load. After flowing through the external circuit, they are re-introduced into the cell via counter electrode 2 on the back, flowing into the redox electrolyte 3. The redox electrolyte 3 then transports the electrons back to the dye molecules 104.

### EXAMPLES

### Example 1: Synthesis of compounds according to the present invention

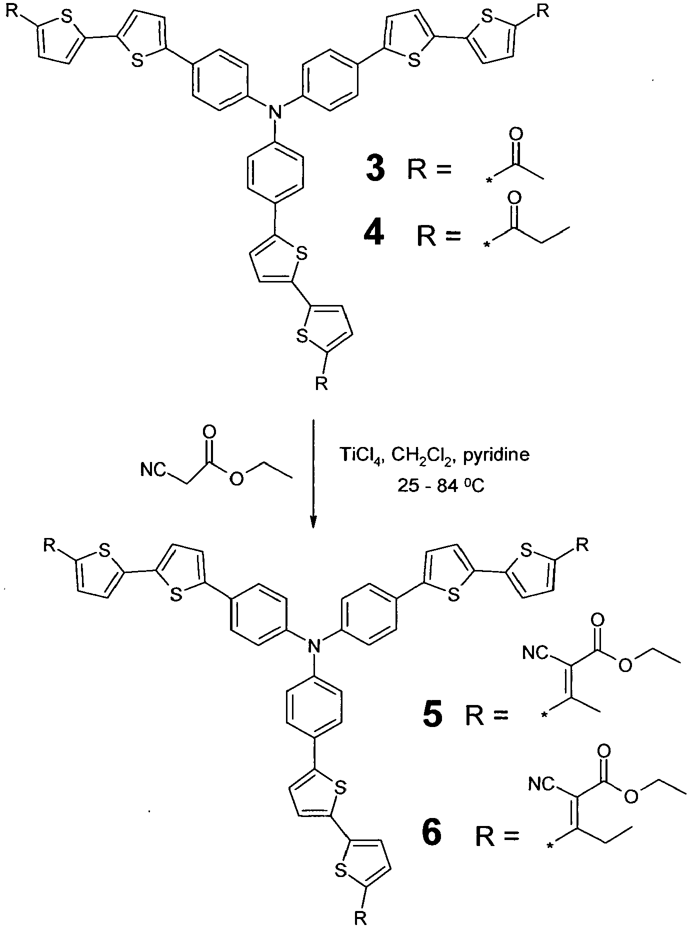

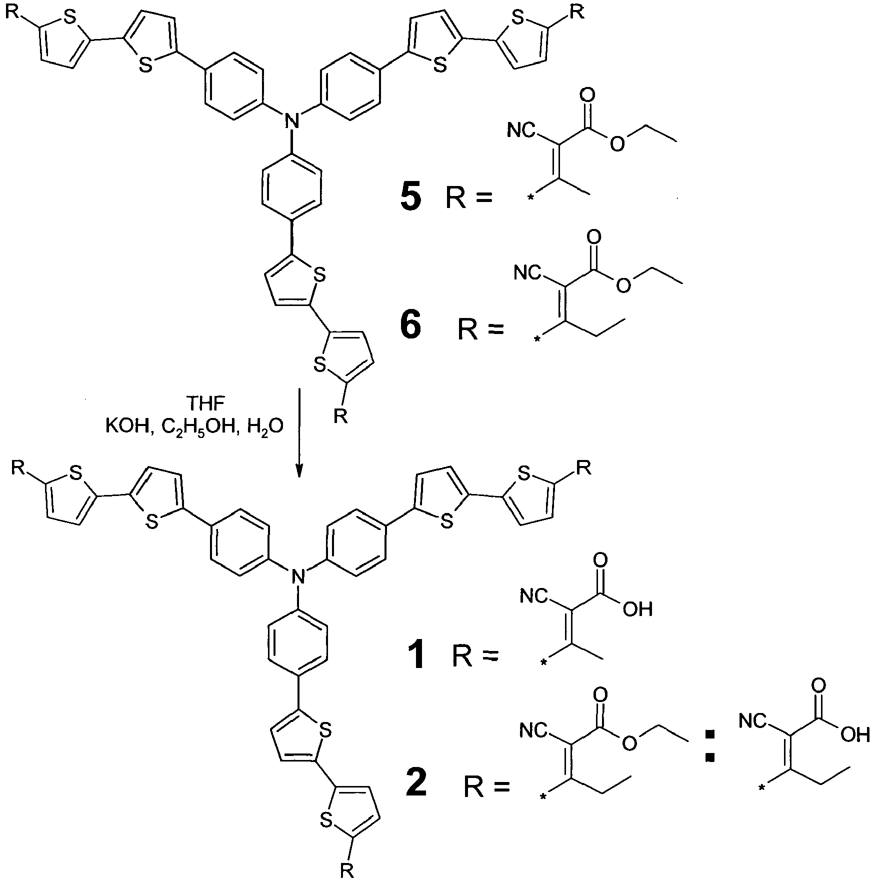

**Synthesis of 5:** Compound **5** was prepared by Knövenagel condensation of **3** and ethylcyanoacetate. Compound **3** (1.35 g, 1.56 mmol) and ethyl cyanoacetate (4.27 g, 37.5 mmol) were dissolved at heating in dry 1,2-dichloroethane (350 ml) and cooled to room temperature. Afterwards, TiCl₄ (1.04 ml, 9.4 mmol) was added dropwise to the reaction mixture and the mixture was stirred at room temperature for 20 min and then the second portion of dry pyridine (3.92 g, 49.7 mmol) was added dropwise. The reaction mixture was stirred at reflux for 3 hours under argon stream. After that, the reaction mixture was filtered and the solution was passed through silica gel column (eluent warm dichloroethane). The solvent was evaporated in vacuum and the residue was dried at 1 Torr to give a crude product, which was purified from the rest of ethylcyanoacetate by precipitation from its solution in THF by addition of hexane. Finally, the crude product was purified by preparative GPC chromatography to give pure 5 (0.49 g, 30%) as a mixture of geometrical isomers. ¹H NMR (250 MHz, CDCl₃, δ, ppm): 1.34 (t, 3H, *J =* 7.3 Hz), 1.38 (t, 6H, *J =* 7.3 Hz), 2.66 (s, 2H), 2.78 (s, 7H), 4.25-4.37 (overlapping peaks, 6H), 7.14 (d, 6H, *J =* 8.5 Hz), 7.20(d, 3H, *J =* 3.7 Hz), 7.24 (d, 3H, *J =* 3.7 Hz), 7.28 (d, 3H, *J =* 4.3 Hz). 7.47 (d, 1 H, *J* = 4.3 Hz). 7.51 (d, 6H, *J* = 8.5 Hz), 7.97 (d, 3H, *J=* 3.7 Hz).

**Synthesis of 1:** The mixture of compound **5** (0.4 g, 0.3 mmol) in THF (40 ml), ethanol (5 ml) and water (5 ml) with KOH (0.33 g, 5.9 mmol) was stirred at reflux for 30 minutes. After that, the equimolar amount of 1 M HCl (5.9 ml) was added dropwise to this mixture and the resulting reaction mixture was diluted with water and filtered. The product on filter was washed with water and dried under reduced pressure to give crude product (0.26 g, 70%) as a mixture of geometrical isomers with different number of hydrolysed groups containing in average 3 COOH-groups.

**Synthesis of 6:** Compound **6** was prepared by Knövenagel condensation of **4** and ethylcyanoacetate. Compound **4** (1.25 g, 1.38 mmol) and ethyl cyanoacetate (3.76 g, 33.1 mmol) were dissolved at heating in dry 1,2-dichloroethane (350 ml) and cooled to room temperature. TiCl₄ (0.92 ml, 8.3 mmol) was added dropwise to the reaction mixture and the mixture was stirred at room temperature for 20 min and then second portion of dry pyridine (3.92 g, 49.7 mmol) was added dropwise. The reaction mixture was stirred at reflux for 45 min under argon stream. After that, the reaction mixture was filtered and the solution was passed through silica gel column (eluent warm dichloroethane). The solvent was evaporated in vacuum and the residue was dried at 1 Torr to give a crude product, which was purified from the rest of ethylcyanoacetate by precipitation from its solution in THF by addition of hexane. Finally, the crude product was purified by preparative GPC chromatography to give pure 6 (0.6 g, 37%) as a mixture of geometrical isomers. ¹H NMR (250 MHz, CDCl₃, δ, ppm): 1.08-1.32 (overlapping triplets, 18H), 2.89 (m, 2H), 3.09 (m, 4H), 4.15-4.32 (overlapping peaks, 6H), 7.08 (d, 6H,), 7.41(d, 1H,), 7.44-7.56 (m, 8H,). 7.58 (d, 1H), 7.65 (d, 6H), 7.97 (d, 2H). ¹³C NMR (600MHz, DMSO-d6, δ, ppm): 14.16, 14.38, 14.81, 24.02, 28.79, 29.36, 33.04, 62.23, 62.52, 67.08, 67.48, 98.08, 101.05, 107.45, 116.6, 118.13, 124.67, 124.77, 125.03, 125.37, 127.15, 127.74, 128.15, 128.37, 128.46, 133.91, 135.14, 135.27, 135.49, 137.42, 143.74, 144.42, 143.74, 144.42, 144.83, 146.57, 162.43, 162.92, 165.43.

**Synthesis of 2:** The mixture of compound **6** (0.45 g, 0.4 mmol) in THF (50 ml), ethanol (10 ml) and water (10 ml) with KOH (0.36 g, 6.4 mmol) was stirred at room temperature for 40 minutes. After that, the equimolar amount of 1 M HCl (6.4 ml) was added dropwise to this mixture and the resulting reaction mixture was diluted with water and filtered. The product on filter was washed with water and dried under reduced pressure to give crude product (0.37 g, 87%) as a mixture of geometrical isomers with different number of hydrolysed groups containing 1-3 COOH groups. ¹³C NMR (600MHz, DMSO-d6, δ, ppm): 8.83, 14.16, 14.24, 14.38, 14.81, 15.12, 25.6, 28.79, 31.73, 62.23, 62.53, 63.23, 67.48, 98.11, 107.42, 118.15, 118.59, 120.78, 123.6, 124.7, 124.91, 125.08, 125.29, 125.38, 125.81, 126.99, 127.15, 127.74, 127.96, 128.16, 128.43, 133.9, 134.48, 134.96, 135.14, 135.27, 135.51, 137.42, 141.83, 143.74, 144.39, 144.48, 144.85, 146.58, 146.65, 162.43, 165.45, 193.62.

### Example 2: Preparation of an DSSC according to the present invention

A solar cell was manufactured similar to the procedure in Example 3 of EP-A-2 386 607. A TiO₂ paste (Solaronix, Ti-Nanoxide T/SP) was screen printed on a fluorine-doped tin oxide glass substrate to form a TiO₂ transparent layer with a thickness of 10 µm. The TiO₂ electrode was treated with a 40 mM TiCl₄ solution at 70 °C for 30 minutes, and sintered at 500 °C for 30 minutes. Subsequently, the TiO₂ electrode was respectively impregnated with compound 5 (0.3 mM dye in tetrahydrofuran) and then allowed to stand at room temperature for 24 hours. A hot melt film (Surlyn) was positioned as a spacer between the dye-adsorbed TiO₂ electrode and a platinum counter electrode, and heated to 80°C to assemble a sealed sandwich type electrode. As an electrolyte solution, 1,2-dimethyl-n-propylimidazolium iodide (DMPIml) (0.6 M), I₂ (0.1 M), NMBI (0.5 M) and poly(vinylidenefluoride-co-hexafluoro)propylene (PVDF-HFP) (5 wt%) dissolved in 3-methoxypropionitrile (MPN) were used.

### REFERENCE LIST

- 1: first electrode
- 101: substrate of the first electrode (for example glass)
- 102: transparent conductive coating (for example ITO)
- 103: porous oxide semiconductor layer (for example based on TiO₂-particles)
- 104: compound according to the present invention
- 2: second electrode (counter electrode)
- 3: redox electrolyte

## Claims

1. Compounds of the general formula (I) 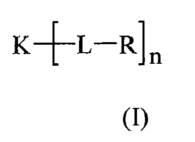 wherein
n is an integer from 3 to 6,
R represents H or a non-conjugated chain,
L are linear conjugated units according to the general formula (II) 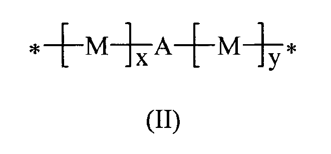 in which
x, y in each case independently of each other represent an integer from 0 to 20,
A represents an acceptor group selected from the group consisiting of group IIIa-IIIl 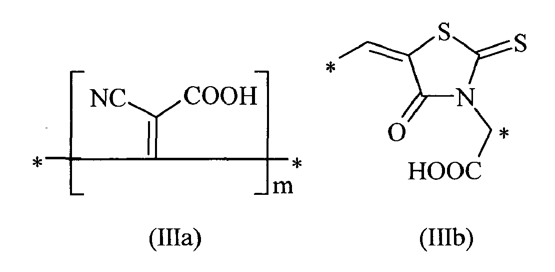 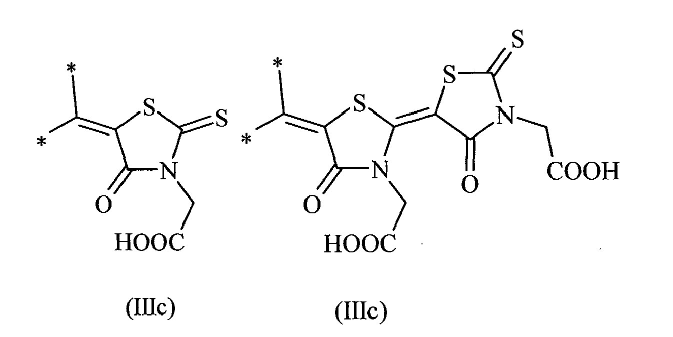 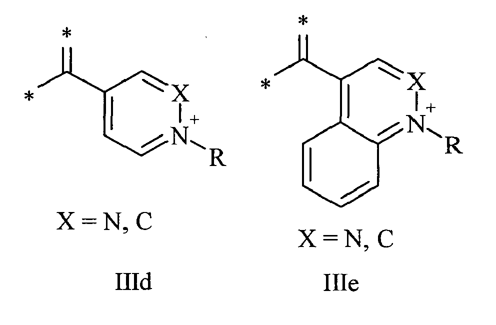 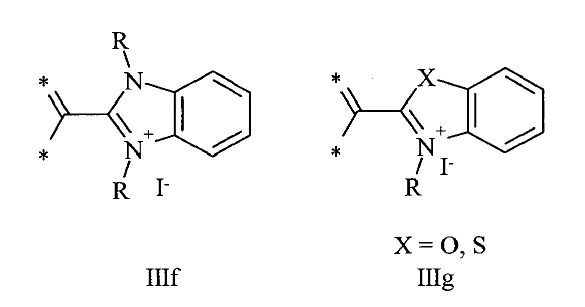 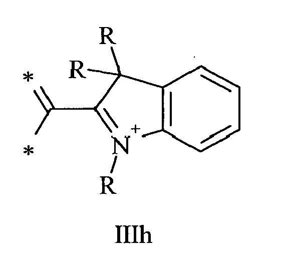 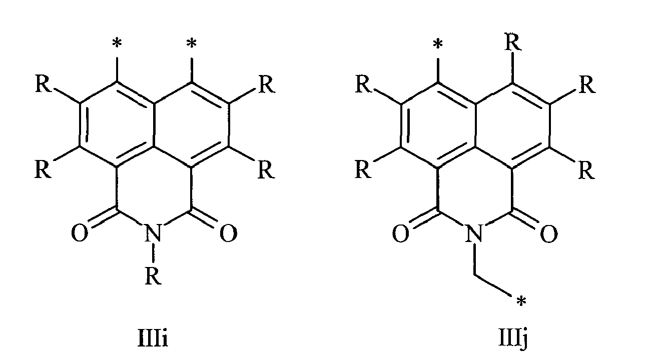 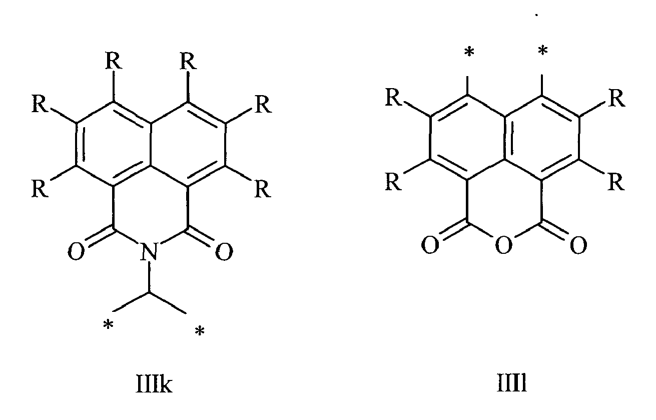 in which
R represents H or a linear or branched C₁-C₂₀-alkyl group, preferably a C₁-C₁₂-alkyl group, or a linear C₁-C₂₀-alkyl group, preferably C₁-C₁₂-alkyl group, which is optionally interrupted by one or more O or S atoms or silylene, phosphonoyl or phosphoryl groups, where, if the aryl compound A comprises two radicals R³, these can be identical or different and
m represents an integer from 1 to 20,
and wherein in case of group IIIa the NC-group may be partially or completely hydrolyzed to form a H₂N-C(O)-group or a OH-C(O)-group and the -COOH-group may be esterified to form a-COOR¹-group, in which R¹ represents a C₁-C₁₀-alkyl group,
M represents an aryl compound according to one of the following formulae IVa-IVq 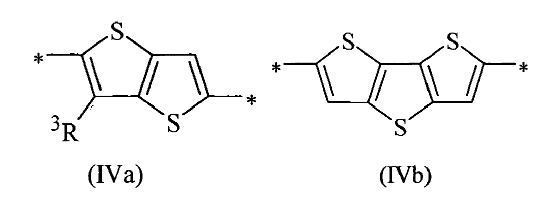 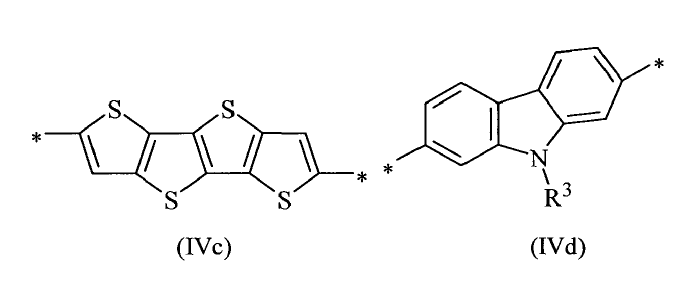 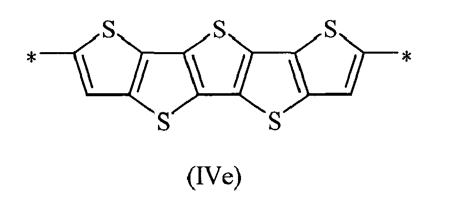 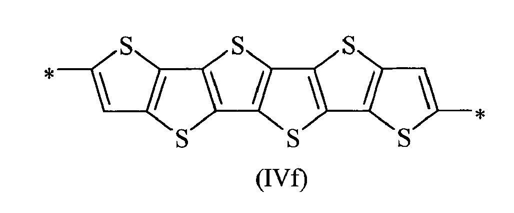 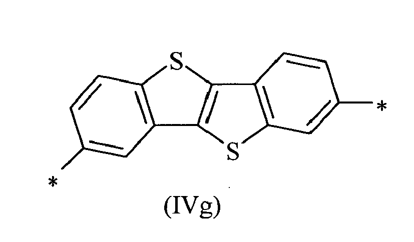 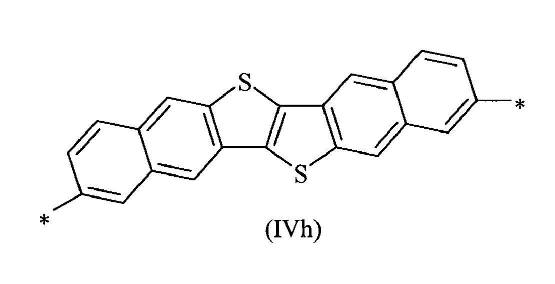 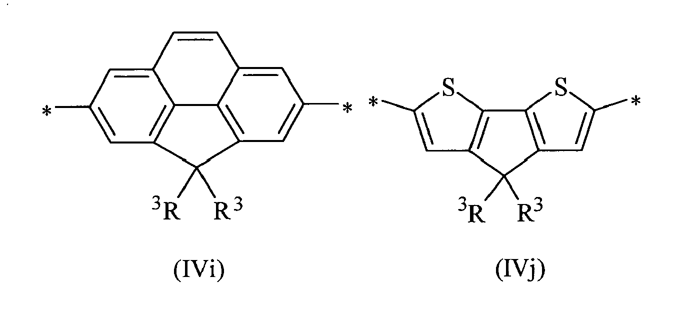 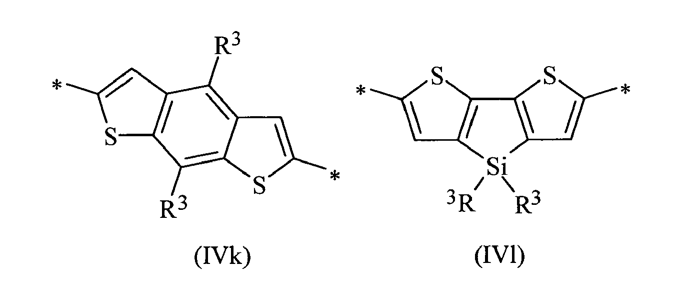 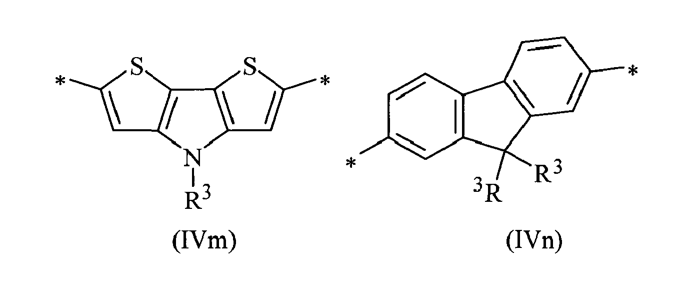 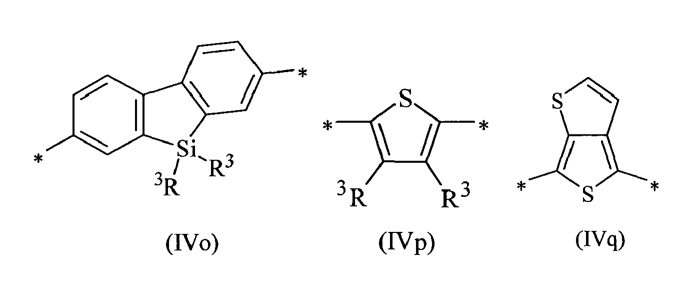 in which
R³ represents H or a linear or branched C₁-C₂₀-alkyl group, preferably a C₁-C₁₂-alkyl group, or a linear C₁-C₂₀-alkyl group, preferably C₁-C₁₂-alkyl group, which is optionally interrupted by one or more O or S atoms or silylene, phosphonoyl or phosphoryl groups, where, if the aryl compound A comprises two radicals R³, these can be identical or different,
K represents a branching group according to one of the following formulae Va-Vi 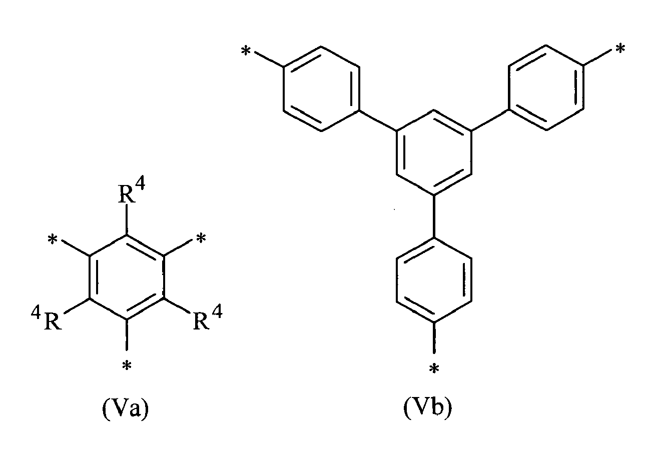 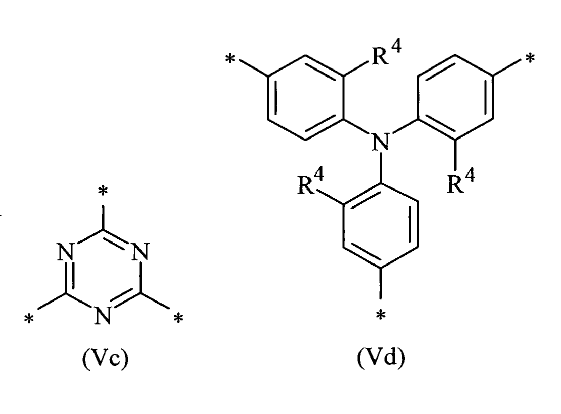 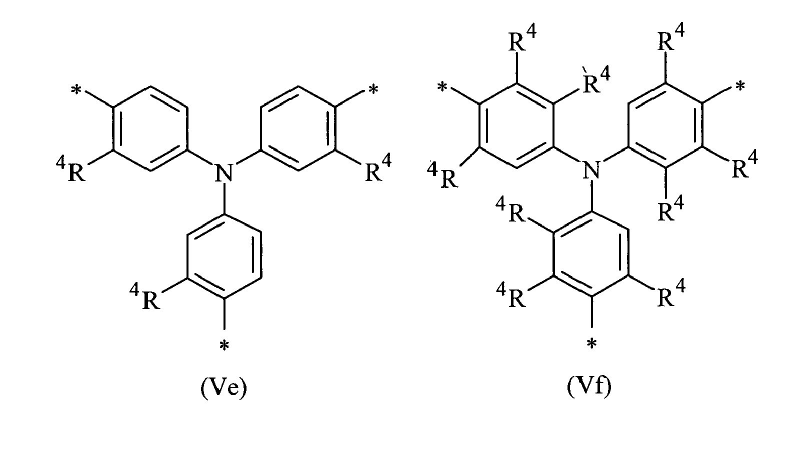 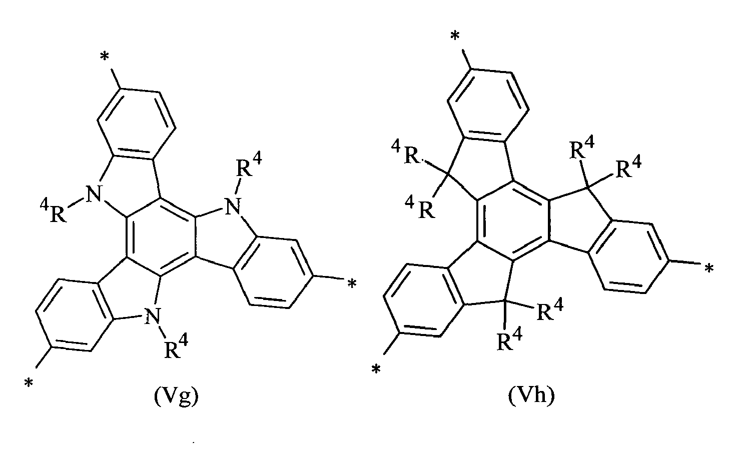 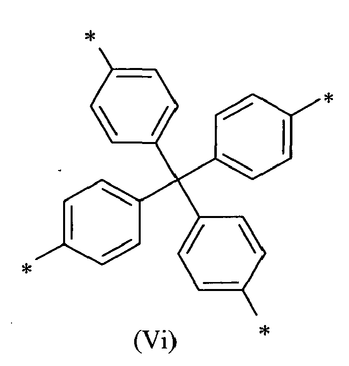 in which
R⁴ represents H or a linear or branched C₁-C₂₀-alkyl group, preferably a C₁-C₁₂-alkyl group, or a linear C₁-C₂₀-alkyl group, preferably C₁-C₁₂-alkyl group, which is optionally interrupted by one or more O or S atoms or silylene, phosphonoyl or phosphoryl groups.

2. Compounds according to claim 1, wherein M represents optionally substituted 2,5-thienylene of formula (IVp): 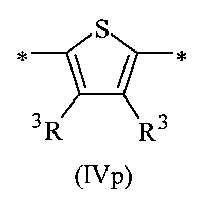 in which
R³ can be identical or different and represents H or a linear or branched C₁-C₂₀-alkyl group, preferably a C₁-C₁₂-alkyl group, or a linear C₁-C₂₀-alkyl group, preferably C₁-C₁₂-alkyl group, which is optionally interrupted by one or more O or S atoms or silylene, phosphonoyl or phosphoryl groups.

3. Compounds according to claim 2, wherein R³ represents a hydrogen atom.

4. Compounds according to claim 3, wherein K represents a branching group of the formula (Vd) 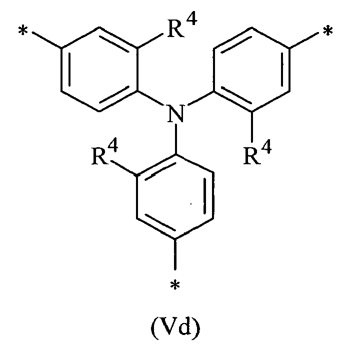 in which
R⁴ represents H or -O-(CH₂)ₙCH₃, wherein n is an integer from 0 to 11.

5. Compounds according to claim 5, wherein:
A represents an acceptor group of formula (IIIa) 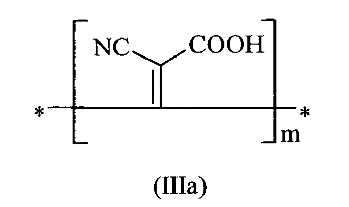
in which m represents 1 and wherein the NC-group may be partially or completely hydrolyzed to form a H₂N-C(O)-group or a OH-C(O)-group and the -COOH-group may be esterified to form a -COOR¹-group, in which R¹ represents a C₁-C₁₀-alkyl group;
x represents 2;
y represents 0;
R represents a -C₂H₅ or a -CH₃ radical.

6. Electronic component comprising at least one semiconducting layer, wherein the semiconducting layer comprises the compound according to at least one of claim 1 to 5.

7. Electronic component according to claim 6, wherein the electronic component is a dye-sensitized solar cell comprising the compound according to at least one of claim 1 to 5.

8. Electronic component according to claim 7, comprising
(i) a first electrode (1) having a porous oxide semiconductor layer (103) coated onto a transparent conductive substrate (101,102), wherein the compound according to the present invention (104) is immobilized on the porous oxide semiconductor layer (103),
(ii) a second electrode (2) which is provided so as to face the porous oxide semiconductor layer (103) of the first electrode (1),
(iii) a redox electrolyte (3) disposed between the first electrode (1) and the second electrode (2).

9. The electronic component according to claim 8, wherein the porous oxide semiconductor layer comprises TiO₂-particles.

10. The electronic component according to claim 9, wherein the compound according to at least one of claims 1 to 5 is bound to the TiO₂-particles via the COOH-group of the acceptor group.

11. Use of compounds according to at least one of claims 1 to 5 for the production of semiconducting layers in electronic components.

12. Use according to claim 11, wherein the electronic component is a dye-sensitized solar cell and wherein the compounds are used as the dye.
